# EUROPEAN PATENT APPLICATION

(11) **EP 4 659 799 A1**
(43) Date of publication of application: **10.12.2025**
(21) Application number: 24750467.3
(22) Date of filing: 19.01.2024
(51) Int. Cl.: A61N 5/06, A61N 5/067, A61N 7/02, A61B 8/00, A61N 7/00

(54) **HANDPIECE FOR OPTICAL TREATMENT, AND TREATMENT METHOD USING SAME**

(30) Priority: 30.01.2023 KR 20230011735
(71) Applicant: Daegu Gyeongbuk Institute of Science and Technology, Daegu 42988 (KR)
(72) Inventor: CHANG, Jinho, Seoul 07997 (KR); KIM, Haemin, Incheon 21325 (KR); KIM, Jin Woo, Dalseong-gun Daegu 43008 (KR)
(74) Representative: dompatent
(86) International application number: PCT/KR2024/000948
(87) International publication number: WO 2024/162665

(57) **Abstract**

The present disclosure provides a handpiece for optical treatment. In one embodiment, the handpiece for optical treatment comprises: a cylindrical body that can be held by a user; an ultrasonic generation part provided at one end of the body so as to emit ultrasonic waves at a treatment area, thereby raising the temperature of the treatment area to a set temperature; a laser emission part which is provided within the body and which emits a laser at the treatment area; and a housing which is coupled to the front end of the ultrasonic generation part, and in which a medium for transmitting the ultrasonic waves to the treatment area is accommodated, wherein the ultrasonic generation part can include a frame, which is coupled to the top of the housing and has a through hole through which the laser passes, and an ultrasonic element coupled to the frame.

## Description

### [Technical Field]

The present disclosure relates to a handpiece for optical treatment using ultrasound and a laser, and a treatment method using the same, and more particularly, to a system for treating skin diseases using a handpiece provided with ultrasound and a laser.

### [Background Art]

Laser treatment is generally widely used for skin lesion treatment. Laser treatment methods are used for the treatment of vascular lesions such as congenital nevi, vascular disorders, moles, and age spots, as well as pigmented lesions caused by pigmentation such as melasma and melanin, and even for skin cancer, and the laser is selected for treatment by considering wavelength, pulse duration, penetration depth, etc., according to the characteristics of various types of chromophores that absorb laser energy in skin tissues.

In this case, laser-based treatment is limited in penetrating deep into the skin because the scattering effect of laser energy in skin tissue is significant. As a result, there was a problem that sufficient penetration depth was not obtained. In addition, when the laser is irradiated outside epidermis like a non-invasive procedure, the light energy is significantly attenuated in an epidermal layer, so if the lesion is deep, complete treatment is difficult due to absorption and scattering in other areas even if long-wavelength laser light is irradiated. In order to solve this, when the output of the irradiation light source is increased or the irradiation time is increased, there is a concern that the epidermal layer may be damaged.

Therefore, research is required on a method of treating a lesion by penetrating the laser deep enough into the skin without affecting the surrounding lesion cells.

In addition, when the lesion cells are distributed over a wide area, there is a problem that the size of the treatment device becomes larger in order to find the locations of the lesion cells or treat the lesion cells. Research to solve this problem is also necessary.

### [Disclosure]

### [Technical Problem]

An object of the present disclosure provides a handpiece for optical treatment capable of treating a target lesion located deep in skin that may not be treated with general laser treatment by increasing a temperature of the target lesion and surrounding skin to an appropriate temperature without affecting normal tissues surrounding lesion cells or by generating bubbles from air in a living tissue to minimize scattering of laser energy and then irradiating a laser, and a treatment method using the same.

In addition, another object of the present disclosure provides a handpiece for optical treatment that is a treatment device in the form of a handpiece provided with ultrasound and a laser and increases the convenience of use for a user, and a treatment method using the same.

In addition, still another object of the present disclosure provides a handpiece for optical treatment for finding a location of lesion cells distributed over a wide area or treating lesion cells while minimizing a size of a treatment device, and a treatment method using the same.

Aspects of the present disclosure are not limited to the above-described aspects. That is, other aspects that are not described may be obviously understood by those skilled in the art from the following specification.

### [Technical Solution]

To solve the above-described problems, the present disclosure provides a handpiece for optical treatment. In an embodiment, a handpiece for optical treatment may include: a cylindrical body that is held by a user; an ultrasound generator provided at one end of the body to irradiate ultrasound to a treatment area; a laser irradiator that is provided inside the body and irradiates a laser to the treatment area; and a housing that is coupled to a front end of the ultrasound generator and has a medium for transmitting the ultrasound to the treatment area accommodated therein, in which the ultrasound generator includes: a frame that is coupled to an upper portion of the housing and has a through hole formed through which the laser passes; and an ultrasound element that is coupled to the frame.

The laser irradiator may include: a light source that irradiates the laser to the treatment area through the through hole; a fixed part that is coupled to one end of the light source inside the body; a moving part that is provided so as to be movable along the inside of the body and on which the fixed part is mounted; and a collimating lens that is coupled to a groove formed inside the fixed part and placed on the front end of the light source.

The laser irradiator may include: a light source that irradiates the laser to the treatment area through the through hole; a coupling part that couples the light source and the ultrasound generator so that the light source corresponds to the through hole; and a collimating lens that is coupled to the housing at a position corresponding to the through hole.

The frame may include a concave portion concavely recessed on one surface adjacent to the housing, and the ultrasound element may be provided in the concave portion in a concave shape corresponding to the concave portion and has a through hole formed in a center.

The frame may include: a disk-shaped center portion that has the through hole formed therein and is coupled to a first ultrasound element; and a ring-shaped peripheral portion that is rotatable with respect to the central portion and coupled to a second ultrasound element.

The second ultrasound element may be provided in n pieces (n is a natural number greater than or equal to 1) and disposed to have a central angle of 360/n° with respect to a center point of the central portion.

The first ultrasound element may be an ultrasound element for imaging and the second ultrasound element may be provided as an ultrasound element for thermal therapy.

The ultrasound element may include: a first ultrasound element that has the through hole formed therein and is provide in a ring shape; and a second ultrasound element that surrounds the first ultrasound element and is provided in the ring shape, and the first ultrasound element may be an ultrasound element for thermal therapy and the second ultrasound element may be provided as an ultrasound element for imaging.

The frame may include a concave portion concavely recessed on one surface adjacent to the housing, and the ultrasound element may be attached to an inner side of the concave portion with respect to the through hole.

The ultrasound element may include a first ultrasound element and a second ultrasound element, and the first ultrasound element and the second ultrasound element may be provided as a single element or an array element.

The present disclosure provides a treatment method using the handpiece for optical treatment. In another embodiment, a treatment method using the handpiece for optical treatment may include: after confirming a lesion area, selectively performing, a first mode in which a bubble is generated between a skin surface and the lesion area using ultrasound in a progress area of a laser to minimize scattering of laser energy and increase a penetration depth of the laser, and then the laser is irradiated to the lesion area to treat the lesion area; and a second mode in which a temperature of the lesion area increases using ultrasound in the progress area of the laser to increase the penetration depth of the laser, and then the laser is irradiated to the lesion area to treat the lesion area.

In the first mode, ultrasound of first energy may be generated while the bubble is generated, ultrasound of second energy may be generated while the lesion area is treated, and the second energy may be provided at a lower level than the first energy.

In the second mode, ultrasound of third energy may be generated while the temperature of the lesion area is raised, ultrasound of fourth energy may be generated while the lesion area is treated, and the fourth energy may be provided at a level similar to or lower than the third energy.

### [Advantageous Effects]

According to an embodiment of the present disclosure, by increasing a penetration depth of a laser and then irradiating the laser by increasing a temperature of a target lesion and a surrounding skin to an appropriate temperature without affecting normal tissues around lesion cells, it is possible to treat the target lesion located deep in the skin that may not be treated with general laser treatment.

In addition, according to an embodiment of the present disclosure, by increasing the penetration depth of the laser, and then irradiating the laser by generating bubbles from the air in biological tissues around the lesion cells on a skin surface to minimize scattering of laser energy, it is possible to treat the target lesion located deep in the skin that may not be treated with general laser treatment.

In addition, according to an embodiment of the present disclosure, it is possible to increase the convenience of use by a treatment device in the form of a handpiece provided with ultrasound and a laser.

In addition, it is possible to find a location of a lesion cell distributed over a wide area or to treat the lesion cell while minimizing a size of the treatment device.

Effects of the present disclosure are not limited to the above-described effects, and effects that are not mentioned will be clearly understood by those skilled in the art to which the present disclosure pertains from the present specification and the accompanying diagrams.

### [BRIEF DESCRIPTION OF THE DRAWINGS]

FIG. 1 is a schematic diagram of an optical treatment device according to an embodiment of the present disclosure.
FIG. 2 is a diagram illustrating a handpiece for optical treatment according to an embodiment of the present disclosure.
FIG. 3 is a diagram illustrating an example of a handpiece for optical treatment according to another embodiment of the present disclosure.
FIGS. 4 and 5 are diagrams illustrating an ultrasound generator according to an embodiment of the present disclosure.
FIGS. 6 to 9 each are diagrams illustrating an ultrasound generator according to another embodiment of the present disclosure.
FIGS. 10 and 11 are diagrams sequentially illustrating a treatment method using a handpiece for optical treatment according to an embodiment of the present disclosure.

### [Best Mode]

Embodiments described in this specification may be modified in various different forms, and technologies according to an embodiment are not limited to embodiments described below. In addition, embodiments according to an implementation example are provided so that this disclosure will be thorough and complete, and will fully convey the concept of the present disclosure to those skilled in the art.

Also, the singular forms used in the specification and appended claims are intended to include the plural forms as well, unless the context specifically dictates otherwise.

In addition, numerical ranges as used herein include all possible combinations of lower and upper limits and all values within that range, increments logically derived from the form and width of the defined ranges, all values defined herein, and upper and lower limits of numerical ranges defined in different forms. Unless otherwise defined in the specification of the present disclosure, values out of a numerical range that may occur due to experimental errors or rounding of values are also included in the defined numerical range.

Furthermore, throughout the present specification, unless explicitly described to the contrary, "including" any components will be understood to imply the inclusion of other elements rather than the exclusion of any other elements.

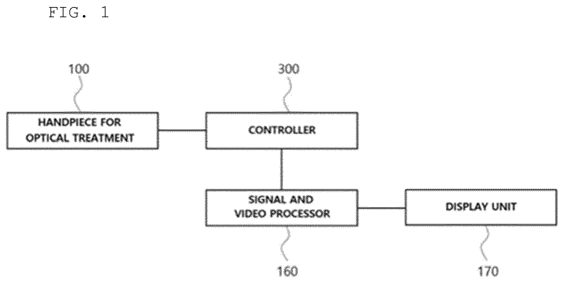

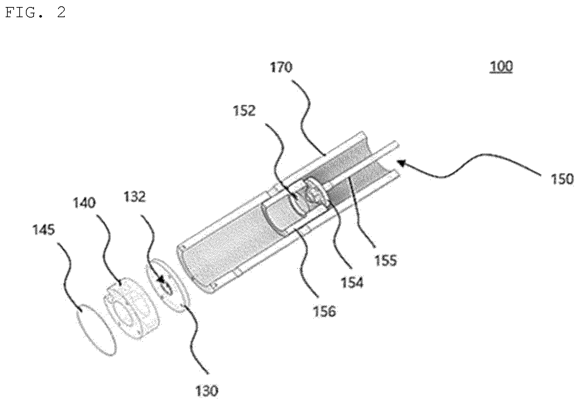

FIG. 1 is a schematic diagram of an optical treatment device 1000 according to an embodiment of the present disclosure, and FIG. 2 is a diagram illustrating a handpiece 100 for optical treatment according to an embodiment of the present disclosure. Referring to FIG. 1, the optical treatment device 1000 includes a handpiece 100 for optical treatment, a signal and video processor 160, a display unit 170, and a controller 300. Referring to FIG. 2, in an embodiment, the handpiece 100 for optical treatment includes a body 120, an ultrasound generator 130, a housing 140, a laser irradiator 150, and a temperature sensor (not illustrated).

In one example, the body 120 is provided in a cylindrical shape so that a user may hold the body 120. The handpiece 100 for optical treatment may be selected by a user by moving the body 120 to select a scan line corresponding to a video area and a heating area.

The signal and video processor 160 generates ultrasound (US) video and photo-acoustic (PA) video of a treatment area. In this case, the signal and video processor 160 may use a video codec to generate a moving continuous image. In addition, the signal and video processor 160 may perform an algorithm that may predict a temperature change in a lesion tissue using the received ultrasound and photo-acoustic signals, and transmit the result to the display unit 170. In this case, the signal and video processor 160 may calculate a temperature change in a treatment area due to radiation of high-intensity focused ultrasound by applying various physical changes according to the temperature change in the treatment area, such as a change in a speckle pattern of the ultrasound video, a change in speed of the ultrasound, or a change in a size of the photo-acoustic signal, to the algorithm.

The display unit 170 receives a signal from the signal and video processor 160 and displays the generated ultrasound or photo-acoustic video on a screen. The display unit 170 may be, for example, a CRT, a liquid crystal display (LCD), a plasma display panel (PDP), an organic light emitting diode (OLED), etc. The user may confirm the lesion area while confirming the ultrasound image through the display unit 170 and move the handpiece 100 for optical treatment.

A temperature sensor (not illustrated) measures the temperature change in the tissue in the treatment area or the surface temperature change in the ultrasound generator 130 and transmits the measured temperature changes to the controller or the display unit.

The controller 300 may control the operation of the laser irradiator 150, a temperature sensor (not illustrated), a transmitting beamformer (not illustrated), the ultrasound generator 130, and a receiving beamformer (not illustrated) to acquire the ultrasound video of the treatment area. In addition, the controller 300 may automatically compensate for the focus of the ultrasound generator 130 confirmed through the display unit 170 or by the user's input. In addition, the controller 300 may control the operation of the ultrasound generator 130 to adjust the temperature of the treatment area to a set temperature.

The housing 140 is coupled to a front end of the ultrasound generator 130 and has a medium, which transmits ultrasound to the treatment area, accommodated inside the housing 140. The housing 140 may be made of a transparent material and may have an ultrasound transmitting medium accommodated inside the housing 140. A laser transmitting area of the housing 140 may be provided as an empty space. For example, a cross section of the housing 140 is provided in a ring shape, and the laser may be transmitted to the lesion through a hole in the center.

The medium provided inside the housing may be provided as a transmitting material with almost no laser energy transmission loss. In one example, the medium may be provided as a transparent material such as water, gel, or gel pad. The medium may be provided in a cylindrical shape with a part open. In one example, the housing 140 may be provided in a form in which one surface is open so that the medium may be filled inside. The film 145 may be provided to allow the open surface of the housing 140 to be opened and closed.

In one example, the laser irradiator 150 includes a light source 155, a fixed part 154, a moving part 156, and a collimating lens 152. The light source 155 irradiates a laser to the treatment area through the through hole 132. The light source 155 is provided at a location corresponding to the through hole 132. In one example, the light source 155 may be provided as an optical fiber, an LED, a laser diode, etc. The light source 155 is not limited thereto, and may have a size that may be positioned within the body 120, but may be provided in various forms. The light source 155 is positioned to be spaced apart from the skin by a certain distance compared to the ultrasound element, so as to prevent the laser from directly contacting the skin and causing damage to the skin.

The fixed part 154 is coupled to one end of the light source 155 inside the body 120. The moving part 156 is provided to be movable along the inside of the body 120 and the fixed part 154 is mounted thereon. As the moving part 156 moves up and down along the inside of the body 120, the light source 155 also moves up and down. Accordingly, the moving part 156 may adjust a focal length of the light source 155. The collimating lens is coupled to a groove formed inside the fixed part 154 and is placed on the front end of the light source 155. The collimating lens allows the laser irradiated from the light source 155 to be progressed uniformly in a parallel direction. The collimating lens allows the laser irradiated from the light source 155 to be progressed uniformly to the treatment area through the through hole 132.

The laser irradiator 150 is provided inside the body 120 and irradiates the laser to the treatment area. In this case, the laser irradiator 150 generates and outputs a laser for thermal therapy or a laser for photoacoustic imaging.

The laser for thermal therapy may be in the form of a continuous wave or a pulsed wave. The generated laser output may be irradiated to a focal point of the treatment area to remove the lesion tissue at the corresponding location. The laser for photoacoustic imaging may be irradiated to an area where a video is to be acquired so that a photoacoustic signal may be generated from the lesion at the corresponding location. In this case, the laser may be a laser of a specific wavelength that allows the lesion tissue to absorb the irradiated energy more than the surrounding normal tissue. In cases where it is difficult to confirm the lesion area with the naked eye, there is an advantage in that the photoacoustic signal generated from the lesion may be confirmed by irradiating the laser for photoacoustic imaging, thereby confirming the lesion area.

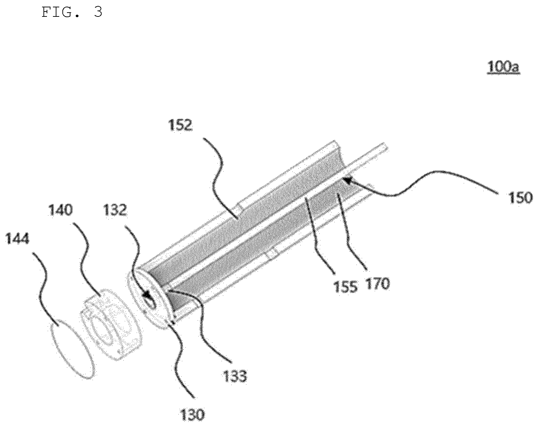

FIG. 3 is a diagram illustrating an example of a handpiece 100a for optical treatment according to another embodiment of the present disclosure. Referring to FIG. 3, in one example, the laser irradiator 150 includes a coupling part 133 and a collimating lens 144. The coupling part 133 couples the light source 155 and the ultrasound generator 130 so that the light source 155 and the through hole 132 correspond to each other, thereby fixing the light source 155 inside the body 120. The collimating lens 144 is coupled to the housing 140 at a position corresponding to the through hole 132. In one example, the housing 140 may be provided as an open cylinder, and the collimating lens 144 may be provided to act as a cover that closes the housing 140 to prevent the medium inside the housing 140 from leaking out. In one example, a diffusion lens may be provided instead of the collimating lens 144. When the diffusion lens is disposed in front of the light source 155, the emitted laser light may be diffused into a wide range of lesion tissues, and thus, when a lesion is formed widely, the treatment is possible efficiently while minimizing the adjustment of the irradiation angle of the light source 155.

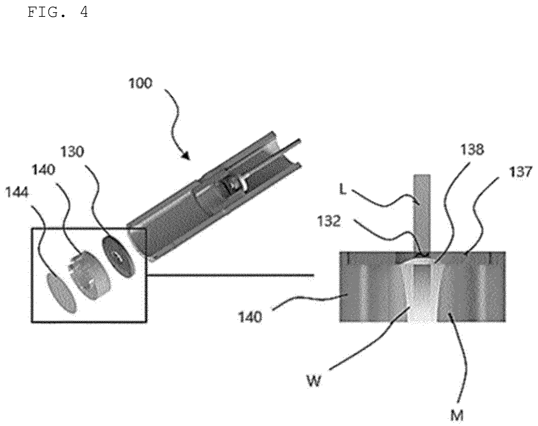

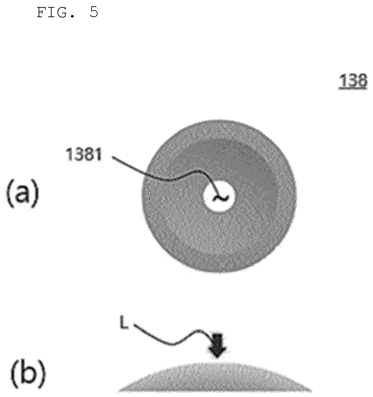

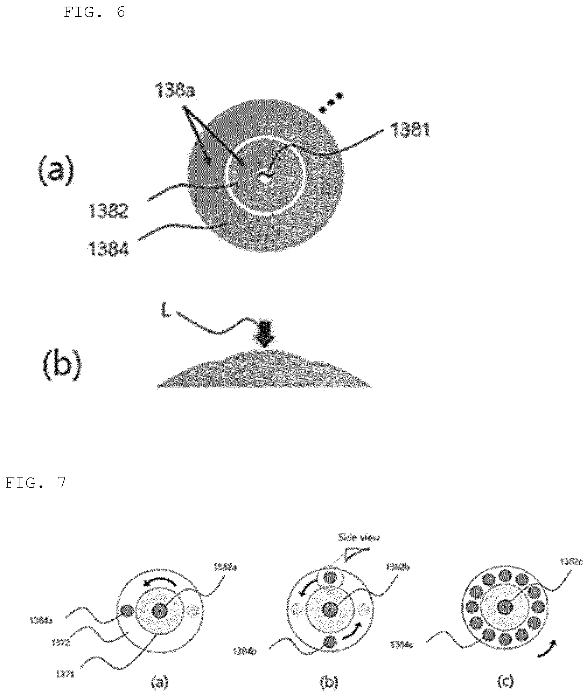

FIGS. 4 and 5 illustrate the appearance of the ultrasound generator 130 according to an embodiment of the present disclosure, and FIGS. 6 to 9 illustrate the appearance of the ultrasound generator 130 according to another embodiment of the present disclosure, respectively. Hereinafter, the ultrasound generator 130 of the present disclosure will be described with reference to FIGS. 4 to 8. Hereinafter, the ultrasound generator 130 according to the present disclosure will be described with reference to FIGS. 4 to 8.

The ultrasound generator 130 transmits ultrasound to the treatment area. The ultrasound generator 130 is provided at one end of the body 120 and irradiates ultrasound to the treatment area. The ultrasound generator 1302 increases the temperature of the treatment area to the set temperature through ultrasound or irradiates the ultrasound to the biological tissue between the skin surface and the surrounding lesion cells to generate bubbles.

Referring to FIG. 4, in one example, the ultrasound generator 130 includes a frame 137 coupled to the upper portion of the housing 140 and provided with a through hole 132 through which a laser passes, and an ultrasound element 138 coupled to the frame 137. FIG. 5 illustrates the appearance of the ultrasound element 138 of FIG. 4. FIG. 5A illustrates a view of the ultrasound element 138 from below, and FIG. 5B illustrates a view of the ultrasound element 138 from the side.

Referring to FIG. 5, in one example, the ultrasound element 138 is provided in a concave shape. A center of curvature of the ultrasound element 138 corresponds to a focus point of the ultrasound beam. In this case, the frame 137 includes a concave portion on one surface adjacent to the housing 140, and the ultrasound element 138 may be provided in a concave shape corresponding to the concave portion within the concave portion. For example, the ultrasound element 138 is provided in a concave shape based on the direction in which the ultrasound is progressed. A through hole 1381 is provided at the center of the ultrasound element 138, and the laser is transmitted to the treatment area through the through hole 1381. The concave portion of the housing 140 and the concave shape of the ultrasound element 138 serve to focus ultrasound into the treatment area. A plurality of ultrasound elements 138 may be attached to the concave portion to facilitate focusing of ultrasound and laser.

Unlike the above, the ultrasound element 138 may be configured as a single element or multiple elements having the form of a linear, convex, or concave array element. In addition, the ultrasound element 138 may be configured as a structure capable of geometric focusing of the laser or a structure capable of electrical focusing by giving an electrical delay to each ultrasound element 138 or using both focusing methods together.

In one example, the ultrasound generator 130 may include a plurality of ultrasound elements 138. For example, the ultrasound element 138 may be composed of an ultrasound element for imaging for acquiring ultrasound video and an ultrasound element for thermal therapy for generating a heating effect. In one example, the ultrasound element for imaging and the ultrasound element for thermal therapy may be separately provided, and the same ultrasound element may be used for both the imaging and thermal therapy purposes.

The ultrasound element for imaging radiates ultrasound to the treatment area to acquire ultrasound video. In one example, a transmitting beamformer (not illustrated) compensates for the focus of the ultrasound of the ultrasound element so that the ultrasound is directed toward the treatment area. A receiving beamformer (not illustrated) receives ultrasound reflected back to the treatment area and the photoacoustic signal generated in the treatment area, and performs dynamic receive beamforming for high-resolution ultrasound and photoacoustic video.

When the ultrasound generator 130 is used for thermal therapy, the ultrasound generator 130 transmits the ultrasound for thermal therapy to increase the temperature of the treatment area. For example, some of the energy of high intensity focused ultrasound (HIFU) generated from the ultrasound element for thermal therapy of the ultrasound generator 130 is transmitted to the target area as heat energy. The treatment area of the focal area is raised to a certain temperature or higher by the heat energy transmitted in this way.

Alternatively, when the ultrasound generator 130 is used for bubble generation, the ultrasound generator 130 transmits ultrasound for bubble generation to generate bubbles. When ultrasound generates bubbles in a path of incident light, scattering of the incident light and defocusing caused by the scattering may be reduced, thereby increasing light penetration. In one example, the ultrasound for bubble generation is provided at intensity sufficiently low enough to avoid damage to skin tissue.

In one example, the ultrasound element may be manufactured from a piezoelectric material having good transmission characteristics, and the ultrasound element for imaging may be manufactured from a material having good transmission/reception characteristics.

FIG. 6 illustrates an ultrasound element 138a according to another embodiment of the present disclosure. FIG. 6A illustrates the ultrasound element 138a viewed from below, and FIG. 6B illustrates the ultrasound element 138a viewed from the side. Referring to FIG. 6, the ultrasound element 138a includes a first ultrasound element 1382 provided in a ring shape with a through hole 132 formed therein, and a second ultrasound element 1384 provided in a ring shape while surrounding the first ultrasound element 1382. In one example, the first ultrasound element 1382 may be the ultrasound element for imaging, and the second ultrasound element 1384 may be provided as the ultrasound element for thermal therapy. The area of the ultrasound element for thermal therapy is provided wider than the area of the ultrasound element for imaging. In addition, the ultrasound for thermal therapy is provided to surround the ultrasound element for imaging. Accordingly, there is an advantage in that the temperature of a desired lesion or bubble generation may be easily increased by using the ultrasound element for thermal therapy.

FIG. 7 illustrates the appearance of the ultrasound element according to another embodiment of the present disclosure. Referring to FIG. 7, the frame 137 may include a disk-shaped central portion 1371 in which the through hole 132 is formed and the first ultrasound element 1382 is coupled, and a ring-shaped peripheral portion 1372 that is provided to be rotatable around the central portion 1371 and to which the second ultrasound element 1384 is coupled. In one example, the frame 137 may form a concave portion by combining the central portion 1371 and the peripheral portion 1372. In another example, the frame 137 may be provided flat. When the frame 137 is provided flat, the ultrasound video may be acquired over a wider area and the ultrasound may be provided.

In one example, the first ultrasound element 1382 may be the ultrasound element for thermal therapy, and the second ultrasound element 1384 may be provided as the ultrasound element for imaging. Accordingly, the ultrasound for thermal therapy may increase the temperature of the treatment area at the center of the through hole 132 to which the laser is irradiated, and the ultrasound element for imaging may rotate near the treatment area to acquire the video of the treatment area. Since the ultrasound element for imaging is provided to be movable, it is easy to find the treatment area. In addition, the ultrasound for thermal therapy may generate bubbles between the skin surface and the lesion at the center of the through hole 132 to which the laser is irradiated, and the ultrasound element for imaging may detect whether bubbles are generated and acquire the video of the treatment area.

In on example, the second ultrasound element 1384 may be provided in n pieces (n is a natural number greater than or equal to 1) and disposed to have a central angle of 360/n° with respect to a center point of the central portion 1371. For example, as illustrated in FIG. 7A, a first ultrasound element 1382a and a second ultrasound element 1384a may be provided as one. Alternatively, as illustrated in FIG. 7B, a first ultrasound element 1382b may be provided as one and a second ultrasound element 1384b may be provided as two. In this case, the second ultrasound element 1384b may be provided to have a central angle of 180° with respect to each other. Alternatively, as illustrated in FIG. 7C, a first ultrasound element 1382c may be provided as one and a second ultrasound elements 1384c may be provided as more than two. The peripheral portion 1372 of FIG. 7C may be provided to be rotatable or fixed.

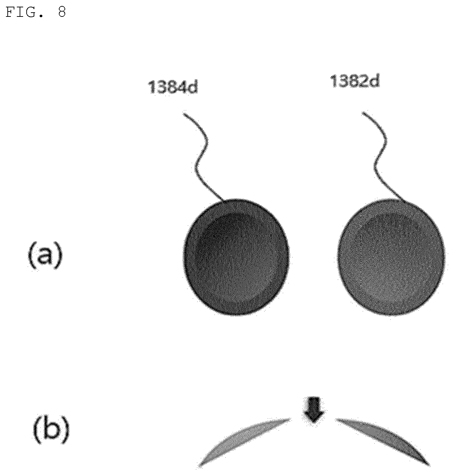

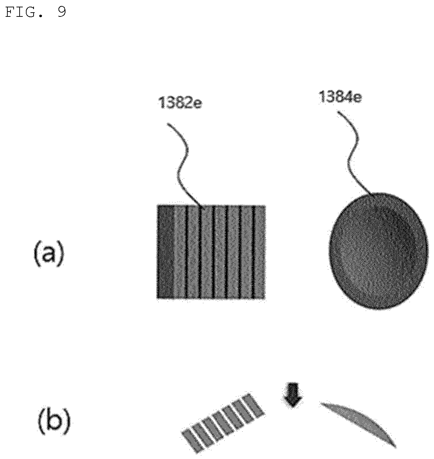

FIGS. 8 and 9 each illustrate the ultrasound element 138 according to another embodiment of the present disclosure. In one example, the frame 137 includes a concave portion on one surface adjacent to the housing 140, and the ultrasound element may be attached to an inner side of the concave portion centered around the through hole 132. In one example, the ultrasound element includes the first ultrasound element 1382 and the second ultrasound element 1384, and the first ultrasound element 1382 and the second ultrasound element 1384 may be provided as a single element or an array element. The first ultrasound element 1382 and the second ultrasound element 1384 each may be provided one by one, or may be provided in greater numbers.

FIG. 8A illustrates a view of the ultrasound element 138 as viewed from below, and FIG. 8B illustrates a view of the ultrasound element 138 as viewed from the side. In one example, the ultrasound element 138 may be disposed to have a central angle of 360/n° with respect to the center of the through hole 132 when viewed from below. In one example, a first ultrasound element 1382d may be the ultrasound element for imaging, and a second ultrasound element 1384d may be the ultrasound element for thermal therapy. Alternatively, both the first ultrasound element 1382d and the second ultrasound element 1384d may be the ultrasound for thermal therapy.

FIG. 9A illustrates a view of the ultrasound element 138 as viewed from below, and FIG. 9B illustrates a view of the ultrasound element 138 as viewed from the side. One of a first ultrasound element 1382e and a second ultrasound element 1384e may be provided as a single element and the other may be provided as an array element. In one example, the first ultrasound element 1382e may be provided as an array element and the second ultrasound element 1384e may be provided as a single element. In one example, the first ultrasound element 1382e may be the ultrasound element for imaging, and the second ultrasound element 1384e may be the ultrasound element for thermal therapy. Alternatively, both the first ultrasound element 1382e and the second ultrasound element 1384e may be the ultrasound for thermal therapy.

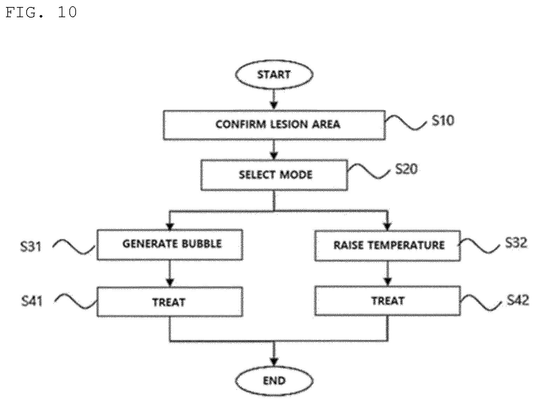

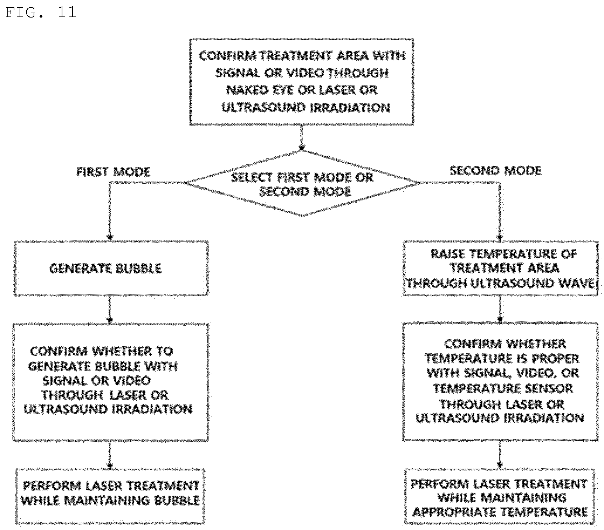

Hereinafter, a treatment method using the handpiece 100 for optical treatment of the present disclosure will be described with reference to FIGS. 10 and 11. The controller 300 controls the operation of the ultrasound generator 130 according to the embodiment of the present disclosure described below.

The ultrasound generator 130 radiates the HIFU from the ultrasound element for thermal therapy to the treatment area to locally raise the temperature of the lesion tissue to a temperature that does not affect the normal tissues around the lesion tissues, and then irradiates a laser of an appropriate wavelength from a light source 155 element to kill the lesion tissue for treatment. Here, when irradiating ultrasound to raise the temperature of the treatment area, the penetration depth of the irradiated laser increases due to the heating effect of the treatment area, and the lesion treatment may be effectively performed in a short period of time.

In addition, the ultrasound generator 130 radiates the HIFU from the ultrasound element for thermal therapy to the treatment area to generate bubbles in the biological tissue between the skin surface and the surrounding lesion cells, thereby minimizing the scattering of laser energy, and then irradiates a laser of an appropriate wavelength to the lesion tissue from the light source 155 element to kill the lesion tissue for treatment. Here, since the scattering of laser energy is minimized in the generated bubbles, the deep lesion treatment may be effectively performed in a short period of time.

Referring to FIGS. 10 and 11, the treatment method using the handpiece 100 for optical treatment of the present disclosure may include a lesion area confirming step (S10), a mode selection step (S20), a bubble generation step (S31), a temperature raising step (S32), and a treatment step (S41 and S42).

In the lesion area confirming step (S10), the lesion area to be treated may be confirmed with the naked eye, or the lesion area may be confirmed through photoacoustic signals, video, etc., by irradiating a laser or ultrasound to an area predicted as the lesion area. When the lesion area is confirmed with the naked eye, the lesion area is confirmed with the naked eye, and the user positions the handpiece 100 for optical treatment so that the laser irradiated through the through hole 132 of the handpiece 100 for optical treatment may reach the lesion area. Alternatively, in cases where it is difficult to confirm the lesion area with the naked eye, the ultrasound video or photoacoustic video is displayed on the screen individually or simultaneously, and the lesion area is confirmed through this.

In the mode selection step (S20), the user selects whether to perform the bubble generation step (S31) or the temperature raising step (S32). In one example, a button may be provided on the handpiece 100 so that the user may select a desired mode, or a means for inputting a mode may be provided on the display unit 170, etc.

When the user selects to perform the bubble generation step (S31) (first mode), the bubble generation step (S31) and the treatment step (S41) are sequentially performed. In the bubble generation step (S31), the ultrasound element irradiates the ultrasound of the first energy to the lesion area. While the ultrasound element irradiates the ultrasound of the first energy to the lesion area, a laser is irradiated from the light source 155.

In one example, the first energy may be ultrasound energy at a level that does not affect living tissue. When ultrasound of relatively low energy is used in the bubble generation step (S31), air bubbles are generated in the ultrasound focused area. When air bubbles are generated in the lesion area, the depth at which the laser penetrates the lesion area may increase as the light energy undergoes Mie scattering in the progress direction due to collision with the air bubbles. In addition, when ultrasound is irradiated onto living tissue, the ultrasound is irradiated at a level that does not affect the living tissue, so the ultrasound does not cause harm to the living tissue.

When bubbles are generated in a desired size and quantity, the treatment step (S41) is performed. In one example, it may be confirmed whether bubbles are generated in a desired size and quantity through the ultrasound video, the photoacoustic video, the ultrasound signal, or the ultrasound video. Alternatively, it may be determined through whether the penetration depth of laser has reached a desired level. Thereafter, in the treatment step (S41), laser light of a wavelength suitable for the lesion tissue characteristics is selected and irradiated to the lesion area. In this case, the ultrasound video and photoacoustic video may be individually or simultaneously used to monitor the treatment process or treatment effect for the lesion area in real time. In one example, in the treatment step (S41), the treatment may be performed so that the bubble generated in the bubble generation step (S31) is maintained. For example, in the treatment step (S41), ultrasound of second energy may be irradiated to the lesion area at the same time as the laser is irradiated to the lesion area. In one example, the second energy may be provided with lower energy than the first energy. For example, the energy for generating the bubble may be provided with higher energy than the energy for maintaining the bubble.

When the user selects to perform the temperature raising step (S32), the temperature raising step (S32) and the treatment step (S43) are performed sequentially. In the temperature raising step (S32), the ultrasound element transmits third energy to the lesion area and its surrounding tissues to raise the surrounding temperature and monitors whether the temperature of the lesion area where the ultrasound is focused has risen to an appropriate temperature. In this step, the ultrasound is used to locally raise the temperature to an appropriate temperature that does not affect the normal tissues around the lesion cells. For this purpose, the third energy may be provided with an energy level that does not affect the normal tissues around the lesion cells. In the temperature raising step (S32), the temperature of the lesion tissue is confirmed using the ultrasound or a separate temperature sensor (not illustrated). In some cases, in addition to confirming the temperature of the lesion tissue, the change in the state of the lesion tissue according to the temperature change may also be monitored. The temperature change of the treatment area may be obtained by applying various physical changes due to the temperature change, such as the change in the speckle pattern of the ultrasound video, the change in speed of the ultrasound, or the change in size of the photoacoustic signals, to a specific algorithm.

When the tissue temperature is raised to an appropriate temperature or higher and sufficient thermal dose is obtained, the constant ultrasound is transmitted to maintain the tissue temperature constant, and at the same time, the treatment step (S42) is performed. In the treatment step (S42), laser light of a wavelength suitable for the lesion tissue characteristics is selected and irradiated to the lesion area. In this case, the ultrasound video and photoacoustic video may be individually or simultaneously used to monitor the treatment process or treatment effect for the lesion area in real time. For example, in the treatment step (S42), ultrasound of fourth energy may be irradiated to the lesion area at the same time as the laser is irradiated to the lesion area. In one example, the fourth energy may be provided with lower energy than the third energy. For example, the energy for raising the temperature may be provided to be greater than energy for maintaining the temperature.

In this way, according to the present disclosure, there is an advantage in that the lesion area may be treated while moving the handpiece 100 for optical treatment that may be held by a user.

In addition, according to the present disclosure, the bubbles may be generated in the skin to be treated or the skin may be raised to an appropriate temperature to generate the heating effect, thereby increasing the penetration depth of the laser. In addition, the treatment is possible with less energy without damaging the surrounding tissue.

In addition, according to the present disclosure, the ultrasound element is provided so that it may rotate near the treatment area and acquire the video of the treatment area. That is, according to the present disclosure, since the ultrasound element for imaging is movably provided, there is an advantage that the ultrasound element for imaging easily finds the treatment area and is applicable to the wide lesion.

As described above, although the present disclosure has been described by detailed components and limited embodiments in the present specification, they have been provided only for assisting in the entire understanding of the present disclosure. Therefore, the present disclosure is not limited to the above-described embodiments. Various modifications and changes may be made by those skilled in the art to which the present disclosure pertains from this description.

Therefore, the teachings described in the present specification should not be limited to the above-described embodiments, and the following claims as well as all modified equally or equivalently to the claims are intended to fall within the scopes and teachings of the specification.

## Claims

1. A handpiece for optical treatment, comprising:
a cylindrical body that is held by a user;
an ultrasound generator provided at one end of the body to irradiate ultrasound to a treatment area;
a laser irradiator that is provided inside the body and irradiates a laser to the treatment area; and
a housing that is coupled to a front end of the ultrasound generator and has a medium for transmitting the ultrasound to the treatment area accommodated therein,
wherein the ultrasound generator includes:
a frame that is coupled to an upper portion of the housing and has a through hole formed through which the laser passes; and
an ultrasound element that is coupled to the frame.

2. The handpiece of claim 1, wherein the laser irradiator includes:
a light source that irradiates the laser to the treatment area through the through hole;
a fixed part that is coupled to one end of the light source inside the body;
a moving part that is provided so as to be movable along the inside of the body and on which the fixed part is mounted; and
a collimating lens that is coupled to a groove formed inside the fixed part and placed on the front end of the light source.

3. The handpiece of claim 1, wherein the laser irradiator includes:
a light source that irradiates the laser to the treatment area through the through hole;
a coupling part that couples the light source and the ultrasound generator so that the light source corresponds to the through hole; and
a collimating lens that is coupled to the housing at a position corresponding to the through hole.

4. The handpiece of claim 1, wherein the frame includes a concave portion concavely recessed on one surface adjacent to the housing, and
the ultrasound element is provided in the concave portion in a concave shape corresponding to the concave portion and has a through hole formed in a center.

5. The handpiece of any one of claims 1 to 4, wherein the frame includes:
a disk-shaped center portion that has the through hole formed therein and is coupled to a first ultrasound element;
and
a ring-shaped peripheral portion that is rotatable with respect to the central portion and coupled to a second ultrasound element.

6. The handpiece of claim 5, wherein the second ultrasound element is provided in n pieces (n is a natural number greater than or equal to 1) and disposed to have a central angle of 360/n° with respect to a center point of the central portion.

7. The handpiece of claim 5, wherein the first ultrasound element is an ultrasound element for imaging and the second ultrasound element is provided as an ultrasound element for thermal therapy.

8. The handpiece of claim 4, wherein the ultrasound element includes:
a first ultrasound element that has the through hole formed therein and is provide in a ring shape; and
a second ultrasound element that surrounds the first ultrasound element and is provided in the ring shape, and
the first ultrasound element is an ultrasound element for thermal therapy and the second ultrasound element is provided as an ultrasound element for imaging.

9. The handpiece of claim 1, wherein the frame includes a concave portion concavely recessed on one surface adjacent to the housing, and
the ultrasound element is attached to an inner side of the concave portion with respect to the through hole.

10. The handpiece of claim 9, wherein the ultrasound element includes a first ultrasound element and a second ultrasound element, and
the first ultrasound element and the second ultrasound element are provided as a single element or an array element.

11. A treatment method using the handpiece for optical treatment of claim 1, comprising:
after confirming a lesion area, selectively performing
a first mode in which a bubble is generated between a skin surface and the lesion area using ultrasound in a progress area of a laser to minimize scattering of laser energy and increase a penetration depth of the laser, and then the laser is irradiated to the lesion area to treat the lesion area; and
a second mode in which a temperature of the lesion area increases using ultrasound in the progress area of the laser to increase the penetration depth of the laser, and then the laser is irradiated to the lesion area to treat the lesion area.

12. The treatment method of claim 11, wherein, in the first mode,
ultrasound of first energy is generated while the bubble is generated,
ultrasound of second energy is generated while the lesion area is treated, and
the second energy is provided at a lower level than the first energy.

13. The treatment method of claim 11, wherein, in the second mode,
ultrasound of third energy is generated while the temperature of the lesion area is raised,
ultrasound of fourth energy is generated while the lesion area is treated, and
the fourth energy is provided at a level similar to or lower than the third energy.
